# EUROPÄISCHE PATENTSCHRIFT

(11) **EP 2 112 888 B1**
(45) Veröffentlichungstag und Bekanntmachung des Hinweises auf die Patenterteilung: **22.06.2016**
(21) Anmeldenummer: 08706786.4
(22) Anmeldetag: 22.01.2008
(51) Int. Cl.: A23J 3/00, A23J 3/14, A23L 27/60, A23L 33/00, A23L 23/00

(54) **WASSERHALTIGES PFLANZLICHES PROTEINPRÄPARAT UND VERFAHREN ZUR HERSTELLUNG DESSELBEN**
AQUEOUS PLANT PROTEIN PREPARATION AND METHOD FOR PRODUCING THE SAME
PRÉPARATION AQUEUSE À BASE DE PROTÉINES VÉGÉTALES ET PROCÉDÉ DE PRODUCTION

(30) Priorität: 23.01.2007 DE 102007003263
(43) Veröffentlichungstag der Anmeldung: 04.11.2009
(73) Patentinhaber: Prolupin GmbH, 18507 Grimmen (DE)
(72) Erfinder: MÜLLER, Klaus, 85356 Freising (DE); EISNER, Peter, 85354 Freising (DE); SCHOTT, Michael, 85416 Langenbach (DE); BEZ, Jürgen, 80687 München (DE); BADER, Stefanie, 85354 Freising (DE); HASENKOPF, Katrin, 85354 Freising (DE); FRANKL, Michael, 81241 München (DE)
(74) Vertreter: Witte, Weller & Partner Patentanwälte mbB
(86) Internationale Anmeldenummer: PCT/DE2008/000112
(87) Internationale Veröffentlichungsnummer: WO 2008/089735

(56) Entgegenhaltungen:
- EP-A- 1 405 572
- WO-A-99/11143
- WO-A-2004/086882
- US-A1- 2005 003 061
- KING J ET AL: "FUNCTIONAL PROPERTIES OF LUPIN PROTEIN ISOLATES (LUPINUS ALBUS CV MULTOLUPA)" JOURNAL OF FOOD SCIENCE, INSTITUTE OF FOOD TECHNOLOGISTS, CHICAGO, IL, US, Bd. 50, Nr. 1, 1. Januar 1985 (1985-01-01), Seiten 82-87, XP002068531 ISSN: 0022-1147

## Beschreibung

### Anwendungsgebiet

Die Erfindung betrifft ein wasserhaltiges pflanzliches Proteinpräparat, vorzugsweise aus Lupinenprotein, ein Verfahren zur Herstellung desselben sowie den Einsatz des Präparates in Lebensmitteln.

### Stand der Technik

Die Verwendung von pflanzlichen Proteinen als Zutat für die Herstellung von Lebensmitteln als Ersatz von tierischen Rohstoffen wie Eiprotein oder Milchprotein gewinnt zunehmend an Bedeutung. Pflanzenproteine zeigen bei einer Vielzahl von Lebensmittelanwendungen zur Ausbildung der Textur sehr gute techno-funktionelle Eigenschaften. Proteinpräparate aus Rohstoffen wie Soja, Reis, Weizen, Erbse, Lupine oder anderen proteinhaltigen Pflanzensaaten kommen in Lebensmitteln zum Beispiel als Wasserbinder, Ölbinder, Gelbildner, Emulgator oder Schaumbildner zum Einsatz.
US 2005/0003061 A1 offenbart ein Verfahren zur Verarbeitung von Lupinenmehl. WO 2004/086882 A1 offenbart ein stabiles puderartiges Produkt mit einem fettlöslichen aktiven bestandteil in einer Matrix, bestehend aus nativen Lupinenproteinen. EP 1 405 572 A1 offenbart ein wässriges Proteingemisch, wobei das Gemisch einen Wassrgehalt von >60 Gew.-% aufweist und durch Zugabe von Protease die enthaltenen Proteine zwischen 1-30% in gespaltenem (hydrolysiertem) Zustand vorliegen. XP 2068531A offenbart die funktionellen Eigenschaften von Lupinenproteinisolaten. WO 99/11143A1 offenbart funktionelle Lupinenproteinisolate und Lupinenproteinkonzentrate.

Um einen mikrobiologischen Verderb weitgehend auszuschließen, werden die Proteinpräparate nach Stand der Technik am Markt in trockener, pulvriger Form angeboten. Bekannt sind Mehle mit einem Proteingehalt <50%, Konzentrate mit einem Proteingehalt >60% und Isolate mit einem Proteingehalt >90%. Die pulverförmigen Zutaten haben in der Regel eine Partikelgröße kleiner 300 µm und neigen beim Einrühren in Wasser zur Bildung von Agglomeraten, was unerwünscht ist und eine lange Zeit für das Einrühren in Anspruch nimmt.

Zur Vermeidung der Fettoxidation, dem sog. Ranzigwerden, kommen für die Herstellung der Proteinpräparate vorzugsweise entfettete Rohstoffe zum Einsatz. Die dabei erhaltenen Proteinpräparate haben einen Fettgehalt kleiner 2% (Bestimmungsmethode: Soxhlet) und bilden somit im Verlauf der Lagerung nur geringe Mengen an Fettoxidationsprodukten aus. Der Nachteil einer weitgehenden Entölung liegt in den hohen Kosten des Verfahrens. Es werden teure Chemikalien wie Hexan benötig und es fallen für die Ausstattung der Anlagen im Explosionsschutzbetrieb hohe Kosten an.

Eine weitere Möglichkeit, das Ranzigwerden von Proteinpräparaten auch bei Fettgehalten größer 2% zu reduzieren, bietet die thermische Behandlung, das sog. Toasten. Hierbei wird die ganze oder zerkleinerte Saat trocken oder angefeuchtet zum Teil unter Druck auf Temperaturen bis teilweise über 120 °C erhitzt, wodurch die saateigenen Enzyme inaktiviert und spätere enzymkatalysierte Fettoxidationsreaktionen vermieden werden.

Die trocken- oder feucht-thermische Behandlung führt allerdings zu einer erheblichen Schädigung der Proteine und damit zu einem Rückgang der Techno-Funktionalität der Präparate. Die Präparate lösen sich schlecht in Wasser oder in der Lebensmittelmatrix auf und verursachen somit ein raues Mundgefühl. In Folge dessen weisen getoastete Präparate nur noch für minderwertige Anforderungen ein ausreichendes techno-funktionelles Profil auf.

Die Aufgabe der vorliegenden Erfindung besteht darin, ein pflanzliches Proteinpräparat zur Verfügung zu stellen, das über sehr gute techno-funktionelle Eigenschaften verfügt, einfach zu verarbeiten ist und eine weitgehende Stabilität gegenüber Fettoxidation aufweist. Das Präparat sollte als Zutat für die Herstellung von Lebensmitteln und als Ersatz für handelsübliche pflanzliche und tierische Proteinpräparate zum Einsatz kommen.

### Darstellung der Erfindung

Die Aufgabe wird mit dem Proteinpräparat und dem Verfahren gemäß den Ansprüchen 1 und 7 gelöst. Vorteilhafte Ausgestaltungen des Präparates sowie des Verfahrens sind Gegenstand der Unteransprüche oder lassen sich der nachfolgenden Beschreibung entnehmen.

Das erfindungsgemäße Proteinpräparat weist im Gegensatz zu herkömmlichen Proteinpräparaten einen Wassergehalt größer als 75 Gew.-% auf. Die im Produkt enthaltene Feststoffmasse enthält mehr als 70 Gew.-% Pflanzenprotein, besonders vorteilhaft >80 Gew.-%. Die Proteine liegen im erfindungsgemäßen Präparat im Wasser zu einem nur geringen Anteil von ≤ 25 % (bezogen auf die Proteine) denaturiert vor. Das bedeutet, dass ein Teil der Proteine eine aufgefaltete Struktur aufweist. Diese denaturierte oder teilweise denaturierte Struktur kann beispielsweise durch Temperatureinwirkung >65°C über einen Zeitraum von 0,1 bis 15 Minuten erzielt werden. Die Kombination aus der Höhe der Temperatur und der Zeit muss dabei so gewählt werden, dass eine stärkere Denaturierung vermieden wird. Aus dem gleichen Grund müssen auch extreme pH-Werte von < 3 der von > 9 vermieden werden. Vorteilhaft enthält der restliche Anteil der Trockensubstanz des Produktes, gegebenenfalls mit Ausnahme von unlöslichen Faserstoffen, Fett bzw. Öl aus der Saat. Der Anteil unlöslicher Fasern liegt vorzugsweise unter 10 Gew.-% bezogen auf die Trockensubstanz. Dadurch ist eine Beeinträchtigung des Mundgefühls durch unlösliche Fasern weitgehend ausgeschlossen.

Die Festlegung des Proteingehaltes in Gew.-% in der vorliegenden Anmeldung erfolgt durch Analyse des Stickstoffgehaltes in Gew.-% und Multiplikation dieses Wertes mit dem Faktor 6,25.

Das erfindungsgemäße Proteinpräparat liegt als wässriges Gemisch vor, insbesondere als eine homogene Proteinsuspension oder eine Lösung. Die Viskosität dieses Proteinpräparates liegt bei einer Scherrate von 300 l/s und einer Temperatur von 20 °C vorteilhaft über 10 mPas.

Das erfindungsgemäße Proteinpräparat hat eine Reihe von Vorteilen gegenüber den am Markt verfügbaren trocknen, pulverförmigen Präparaten. So ist das Produkt sehr einfach und nahezu vollständig in Wasser oder wasserhaltigen Lebensmitteln löslich bzw. es lässt sich sehr einfach und gleichmäßig in Wasser einrühren. Benetzungsprobleme, die bei Einsatz von pulverförmigen Präparaten auftreten, gibt es nicht. Dies ermöglicht eine schnelle und einfache Verarbeitung in der Lebensmittelindustrie. Dadurch kann die bislang erforderliche Herstellung einer Protein-Grundsuspension aus Wasser und Proteinpulver entfallen, wodurch Produktionszeit eingespart wird. Die einfache Benetzung und das gleichmäßige Vermischen des erfindungsgemäßen Präparates mit der Lebensmittelmatrix führen im Produkt zu einem verbesserten Mundgefühl.

Durch den hohen Proteingehalt in der Trockensubstanz können auch sensible Lebensmittel hergestellt werden, die den Einsatz von faserhaltigen Inhaltsstoffen ausschließen wie z.B. Mayonnaisen.

Das vorgeschlagene Proteinpräparat liegt in einer vorteilhaften Ausgestaltung als Lösung bzw. als homogene Suspension mit einer Viskosität größer 10 mPas vor. Hinsichtlich der Konsistenz unterscheidet es sich damit nur geringfügig von frischem Eigelb oder homogenisiertem Vollei. Dies hat zur Folge, dass bei der Verarbeitung des Präparates keine Prozessanpassungen im Vergleich zur Verarbeitung von Ei erfolgen müssen. Ein weiterer Vorteil ist, dass dem Lebensmittel-Hersteller das Präparat verarbeitungsfertig bereitgestellt werden kann. So kann das Präparat ohne Vorbereitung einer Vorsuspension wie Flüssigei direkt verarbeitet werden.

Der hohe Proteingehalt in der Trockensubstanz in Kombination mit der Teildenaturierung des Proteins führt zu außergewöhnlich guten techno-funktionellen Eigenschaften des Präparates. So zeigt das Präparat zum Beispiel bei Verwendung von Lupinenprotein im Präparat insbesondere im Bereich der Emulgierkapazität Werte, die zum Teil um 100% über den Werten von handelsüblichen pulvrigen Proteinpräparaten aus Lupine oder Soja liegen. Somit ist die Qualität des im Präparat vorliegenden und teilweise denaturierten Pflanzenproteins im Vergleich zu handelsüblichen Pflanzenproteinen deutlich erhöht.

Ein weiterer Vorteil liegt in der Möglichkeit, das Ranzigwerden des im Präparat enthaltenen Pflanzenöls oder -fettes erheblich verzögern zu können. Dies kann durch Kühlen oder Tiefgefrieren erreicht werden. Besonders im gefrorenen Zustand bleibt das Präparat über viele Monate lagerstabil, ohne dass die Sensorik aufgrund von Fettoxidationsprodukten beeinträchtig wird. Die Stabilität des Präparates bei Temperaturen unter dem Gefrierpunkt zeigt sich überraschenderweise vor allem bei einem hohen Wassergehalt im Präparat. Werden im Vergleich zum erfindungsgemäßen wasserhaltigen Präparat trockene, pulverförmige Präparate bei Temperaturen um oder unter 0°C gelagert, wird die Fettoxidation bei diesen Präparaten im Vergleich zu einer Lagertemperatur von +15°C zum Teil beschleunigt.

Somit zeigt das erfindungsgemäße Proteinpräparat besondere Vorteile hinsichtlich der Lagerstabilität. Auch die mikrobielle Lagerstabilität ist durch Tiefgefrieren gewährleistet.

Dabei zeigt sich zudem, dass das Tiefgefrieren des erfindungsgemäßen Proteinpräparates keinen negativen Einfluss auf die Technofunktionalität hat. So konnte in Versuchen mit einem gefrorenen Lupinenprotein mit 80 % Wasseranteil nach einer Lagerzeit von 12 Monaten noch die gleiche Emulgierkapazität wie vor dem Tiefgefrieren gemessen werden.

Die Herstellung des erfindungsgemäßen Proteinpräparates kann durch folgende Verfahrensschritte erfolgen:
1) Zerkleinern der Samen der als Rohstoff verwendeten Proteinpflanzen, bspw. durch Mahlen oder Flockieren, ggf. nach vorherigem Schälen.
2) Zerkleinerte oder flockierte Samen in der 5 bis 10-fachen Menge Wasser 1-3 mal bei sauren pH-Werten vorextrahieren, vorzugsweise nahe am isoelektrischen Punkt (pH < 5) und/oder bei Temperaturen <25°C, und den Feststoff nach jedem Vorextraktionsschritt mechanisch von der flüssigen Phase trennen.
3) Extrahieren des Pflanzenproteins aus dem vorextrahierten Feststoff mit Wasser bei einem pH-Wert >6,5, vorzugsweise >7,0, und anschießend mechanisches Abtrennen der unlöslichen Bestandteile wie z.B. der Fasern.
4) Ausfällen des gelösten Proteins aus der Lösung durch Zugabe von Säure, vorzugsweise nahe am isoelektrischen Punkt.
5) Abtrennen des gefällten Proteins vom Überstand durch Fest/Flüssig-Trennung nach Stand der Technik. Das gefällte und vom Überstand abgetrennte feuchte Proteinpräparat enthält bspw. ca. 20 bis 25 % Trockenmasse.
6) Nur optional: Neutralisation des ausgefällten Proteins und Einstellung des Trockensubstanz-Gehaltes z.B. auf 15 Gew.-% durch Zugabe von Wasser.
7) Erhitzen des ausgefällten Proteins > 65°C.

Danach hat das Präparat eine erfindungsgemäße Zusammensetzung und kann verpackt und ggf. gekühlt oder tiefgefroren werden.

Es ist vor oder während der Herstellung des erfindungsgemäßen Präparats auch möglich, eine zusätzliche Erhitzung auf Temperaturen >95°C vorzunehmen. Damit können ggf. besondere Effekte hinsichtlich der geschmacklichen oder farblichen Beschaffenheit des Präparats erreicht werden.

Eingesetzt wird das Präparat vorzugsweise als Ersatz für Milchprotein oder Eiprotein oder trocknem Pflanzenprotein in Lebensmitteln. Durch die ähnliche Konsistenz wie flüssiges Eigelb oder flüssiges Vollei können diese Lebensmittelzutaten besonders gut durch das erfindungsgemäße Präparat ersetzt werden.

Das erfindungsgemäße Präparat bietet sich an für Lebensmittel-Emulsionen wie Suppen, Saucen, Süßspeisen, Desserts, Aufstriche, Mayonnaisen, Füllungen von Schokolade oder Backwaren. Auch ein Einsatz für texturbildende oder Fett- bzw. wasserbindende Aufgaben in Wurstwaren, Teigwaren, Backwaren oder feinen Backwaren ist möglich.

Das erfindungsgemäße Präparat enthält Pflanzenproteine aus einem Rohstoff oder Proteinmischungen aus mehreren Rohstoffen. Als Rohstoffe können Ölsaaten zum Einsatz kommen wie beispielsweise Raps, Sonnenblumen, Leinsaat oder anderen Ölsaaten. Auch Proteine aus Leguminosen können im erfindungsgemäßen Präparat enthalten sein. Beispiele sind Soja, Erbse, Lupine, Ackerbohne oder andere. Möglich sind auch weitere pflanzliche Rohstoffe wie Getreide, Reis oder Kartoffeln.

Die Verwendung von Lupinenproteinen bietet besondere Vorteile. So zeigt das erfindungsgemäße Präparat, wenn der Proteinanteil aus Lupinenprotein besteht, neben der Technofunktionalität auch ein ausgeprägtes bioaktives Potenzial zur Cholesterineinstellung beim Menschen. Dies kann für die Herstellung von Functional Food Produkten genutzt werden.

Besondere Vorteile ergeben sich für die Vermarktung des erfindungsgemäßen Präparates. Es kann sehr effizient mit den gleichen Verpackungsformen und Materialien verpackt werden, wie sie für Milchprodukte und Flüssigeiprodukte zum Stand der Technik gehören. Beispiele sind Verpackungsformen wie Verbundverpackungen (z.B. Tetrapak^{®}), Schlauchbeutel, Standbeutel, tiefgezogene Kunststoffbecher und Vakuumbeutel. Dadurch können für das Präparat die gleichen Verpackungsmaschinen zum Einsatz kommen, wie sie aus der Molkereitechnik bekannt sind, was Kosten spart. Die nach Stand der Technik bekannte keimfreie oder aseptische Abfüllung nach einer Pasteurisierung, Sterilisierung oder UHT-Erhitzung ermöglicht eine Haltbarkeit des Präparates von mehreren Wochen im gekühlten Zustand ohne dass das Präparat tiefgefroren werden muss..Es ist auch möglich, das Präparat nach der Abfüllung in der Verpackung z.B. in einem Wasserbad, einem Tunnelpasteur oder in einem Autoklaven zur Herstellung einer Art Dauerkonserve zu erhitzen, wodurch die Mindesthaltbarkeit weiter gesteigert werden kann. Dabei zeigt sich, dass bei Temperaturen von über 110 °C die Emulgierkapazität auch in der Verpackung noch weiter gesteigert werden kann.

Die Möglichkeit der Nutzung von Verpackungsformen wie Tetrapak^{®} oder anderer Milchprodukte-Verpackungen ermöglicht dem Anwender in der Lebensmittelindustrie eine einfache Lagerung und Dosierung. Für größere Produktionsbetriebe können die gleichen Verpackungsgebinde, Container und Darreichungsformen genutzt werden, wie sie aus dem Transport von Flüssigei im industriellen Maßstab bekannt sind.

Im Folgenden wird die Herstellung eines erfindungsgemäßen Proteinpräparates aus Lupinenproteinen anhand eines Ausführungsbeispiels nochmals kurz beschrieben.

10 kg Lupinensamen werden in einer Unterläufer-Schäleinheit geschält. Anschließend werden durch Windsichtung die Schalen vom Kernfleisch abgetrennt. Die schalenfreien Körner werden in einer Flockierwalze zu 0,2 mm starken Plättchen flockiert. Die erhaltenen sog. "Lupinenflakes" werden mit 10 Teilen Wasser, das einen pH-Wert von 4,5 und eine Temperatur von 15 °C hat, in einem Rührbehälter dispergiert. Der Ansatz wird für 60 Minuten konstant bei pH 4,5 und 15 °C gehalten. Danach wird in einem Dekanter die wässrige Phase von der unlöslichen Faserfraktion abgetrennt. Der feuchte Feststoff wird erneut in einem Rührbehälter mit 5 Teilen Wasser (pH-Wert 7,5, Temperatur 35 °C) dispergiert. Nach 45 Minuten wird der bei pH 7,5 und 35 °C konstant gehaltene Ansatz durch Dekantation in einen unlöslichen Feststoff und eine proteinhaltigen Extrakt getrennt. Das im Extrakt enthaltene Protein wird durch Zugabe von Salzsäure auf einen pH-Wert von 4,5 eingestellt, dadurch ausgefällt und durch Separatoren vom wässrigen Überstand abgetrennt. Das ausgefällte Protein wird durch 3 minütiges Erhitzen in einem Röhrenwärmetauscher auf 72 °C pasteurisiert und in Schlauchbeuteln zu je 5 kg abgefüllt und auf 5 °C abgekühlt.
Die gewünschte geringe Denaturierung wird hierbei durch die gewählten geringen Temperaturen bzw. die geringe Zeit bei höherer Temperatur und die gewählten ph-Werte erreicht.

## Patentansprüche

1. Pflanzliches Proteinpräparat, insbesondere aus Lupinenprotein, das als wässriges Gemisch vorliegt,
wobei das wässrige Gemisch einen Wassergehalt > 75 Gew.-% und eine Feststoffmasse aufweist, die mehr als 70 Gew.-% Pflanzenproteine enthält, und wobei die Pflanzenproteine in dem wässrigen Gemisch zu einem Anteil von ≤ 25 % denaturiert vorliegen.

2. Proteinpräparat nach Anspruch 1,
bei dem ein verbleibender Anteil der Feststoffmasse Pflanzenfett oder Pflanzenöl enthält.

3. Proteinpräparat nach Anspruch 1 oder 2,
bei dem ein Anteil unlöslicher Fasern in dem Gemisch < 10 Gew.-% bezogen auf die Feststoffmasse beträgt.

4. Proteinpräparat nach einem der Ansprüche 1 bis 3,
bei dem die Feststoffmasse > 80 Gew.-% Pflanzenproteine enthält.

5. Proteinpräparat nach einem der Ansprüche 1 bis 4,
bei dem das Gemisch eine Viskosität aufweist, die bei einer Scherrate von 300 l/s und einer Temperatur von 20 °C > 10 mPas beträgt.

6. Proteinpräparat nach einem der Ansprüche 1 bis 5 in einer flüssigkeitsdicht abgeschlossenen Verpackung.

7. Verfahren zur Herstellung eines Proteinpräparates nach einem der Ansprüche 1 bis 5 aus Proteinpflanzen,
mit zumindest den folgenden Schritten:
- Zerkleinern von Samen der Proteinpflanzen;
- Vorextrahieren eines Feststoffanteils, indem die zerkleinerten Samen in Wasser einmal oder mehrmals bei sauren pH-Werten gewaschen und der Feststoffanteil jeweils mechanisch von der flüssigen Phase abtrennt wird;
- Extrahieren von Pflanzenproteinen aus dem vorextrahierten Feststoffanteil mit Wasser bei einem pH-Wert >6,5, bei dem die Pflanzenproteine in dem Wasser in Lösung gehen, und mechanisches Abtrennen von unlöslichen Bestandteilen;
- Ausfällen der gelösten Pflanzenproteine aus der Lösung durch Zugabe von Säure;
- Abtrennen der ausgefällten Pflanzenproteine durch Fest/Flüssig-Trennung, wobei ein Anteil von > 70 Gew.-% Pflanzenproteine in der Feststoffmasse erhalten wird;
- Erhitzen der abgetrennten Pflanzenproteine zur Pasteurisierung auf eine Temperatur > 65°C für einen Zeitraum, bei dem der gewünschte Denaturierungsgrad von ≤ 25% nicht überschritten wird ; und
- Zugabe von Wasser zur Bildung eines wässrigen Gemisches vor oder nach der Erhitzung, wobei das wässrige Gemisch durch die Zugabe des Wassers auf einen Trockensubstanz-Gehalt von < 25 Gew.-% eingestellt wird.

8. Verfahren nach Anspruch 7,
bei dem das Vorextrahieren bei einem pH-Wert des Wassers von <5 erfolgt.

9. Verfahren nach Anspruch 7 oder 8,
bei dem das Extrahieren bei einem pH-Wert des Wassers von <7,0 erfolgt.

10. Verfahren nach einem der Ansprüche 7 bis 9,
bei dem das Gemisch in eine Verpackung abgefüllt, die Verpackung flüssigkeitsdicht verschlossen und das Gemisch in der verschlossenen Verpackung auf eine Temperatur von > 110 °C erhitzt wird.

11. Verwendung des Proteinpräparates nach einem der Ansprüche 1 bis 5 als Ersatz für Milchprotein, Eiprotein oder trockenem Pflanzenprotein in Lebensmitteln.

## Claims

1. Plant protein preparation, in particular of lupine protein, in form of an aqueous mixture, wherein the aqueous mixture comprises a water content of > 75 wt-% and a mass of solid matter which comprises more than 70 wt-% of plant proteins, and wherein the plant proteins in the aqueous mixture are denaturated to a proportion of ≤ 25%.

2. Protein preparation of claim 1,
wherein a remaining proportion of the mass of solid matter contains plant fat or plant oil.

3. Protein preparation of claim 1 or 2,
wherein a proportion of insoluble fibers in the mixture is < 10 wt-% with regard to the mass of solid matter.

4. Protein preparation of any of claims 1 to 3,
wherein the mass of solid matter contains > 80 wt-% of plant proteins.

5. Protein preparation of any of claims 1 to 4,
wherein the mixture comprises a viscosity which, at a shear rate of 300 1/s and a temperature of 20°C, is > 10 mPas.

6. Protein preparation of any of claims 1 to 5 in a liquid-tight sealed packaging.

7. Method for the production of a protein preparation of any of claims 1 to 5 from protein plants, comprising at least the following steps:
- reducing of seeds of the protein plants to small pieces;
- pre-extracting of a solid matter fraction by washing the seeds reduced to small pieces in water once or several times at acidic pH-values, and separating each solid matter fraction mechanically from the liquid phase;
- extracting of plant proteins from the pre-extracted solid matter fraction with water at a pH-value of > 6.5, wherein the plant proteins dissolve in the water, and mechanical separation of insoluble components;
- precipitating of the dissolved plant proteins from the solution by the addition of acid;
- separating of the precipitated plant proteins by solid/liquid separation, wherein a proportion of > 70 wt-% of plant proteins is obtained in the mass of solid matter;
- heating of the separated plant proteins for the pasteurization to a temperature of > 65°C for a period of time where the intended degree of denaturation of < 25% will not be exceeded; and
- addition of water for the formation of an aqueous mixture before or after the heating, wherein the aqueous mixture is adjusted to a dry matter content ≤ 25 wt-% by the addition of the water.

8. Method of claim 7, wherein the pre-extracting is performed at a pH-value of the water of < 5.

9. Method of claim 7 or 8,
wherein the extracting is performed at a pH-value of the water of < 7.0.

10. Method of any of the claims 7 to 9, wherein the mixture is filled in to a packaging, the packaging is sealed in a liquid-tight manner, and the mixture in the sealed packaging is heated to a temperature > 110°C.

11. Use of the protein preparation of any of claims 1 to 5 as a substitute of milk protein, egg protein or dry plant protein in food stuff.

## Revendications

1. Préparation à base de protéines végétales, en particulier composée de protéine de lupin, qui se présente en tant que mélange aqueux, selon laquelle le mélange aqueux présente une teneur en eau > 75 % en poids et une masse de matières solides, qui contient plus de 70 % en poids de protéines végétales, et sachant que les protéines végétales sont présentes sous une forme dénaturée dans le mélange aqueux en une proportion ≤ 25 %.

2. Préparation à base de protéines selon la revendication 1,
selon laquelle une proportion restante de la masse de matières solides contient une matière grasse végétale ou une huile végétale.

3. Préparation à base de protéines selon la revendication 1 ou 2,
selon laquelle une proportion en fibres insolubles présente dans le mélange est < 10 % en poids par rapport à la masse de matières solides.

4. Préparation à base de protéines selon l'une quelconque des revendications 1 à 3,
selon laquelle la masse de matières solides contient plus de 80 % en poids de protéines végétales.

5. Préparation à base de protéines selon l'une quelconque des revendications 1 à 4,
selon laquelle le mélange présente une viscosité, qui est > 10 mPas à une vitesse de cisaillement de 300 l/s et à une température de 20 °C.

6. Préparation à base de protéines selon l'une quelconque des revendications 1 à 5 dans un emballage scellé de manière étanche aux fluides.

7. Procédé pour fabriquer une préparation à base de protéines végétales selon l'une quelconque des revendications 1 à 5 à partir de plantes protéagineuses, comprenant au moins les étapes qui suivent consistant à :
- broyer des graines des plantes protéagineuses ;
- extraire au préalable une proportion en matières solides en ce que les graines broyées sont lavées à l'eau une fois ou à plusieurs reprises à des valeurs de pH acide et que la proportion en matières solides est respectivement séparée mécaniquement de la phase liquide ;
- extraire des protéines végétales de la proportion en matières solides extraite au préalable avec de l'eau à une valeur de pH > 6,5, étape au cours de laquelle les protéines végétales se dissolvent dans l'eau, et séparer mécaniquement des composants insolubles ;
- précipiter les protéines végétales dissoutes à partir de la solution en ajoutant de l'acide ;
- séparer les protéines végétales précipitées par une séparation solide/liquide, sachant qu'une proportion d'une valeur > 70 % en poids de protéines végétales est contenue dans la masse de matières solides ;
- chauffer les protéines végétales séparées aux fins de la pasteurisation à une température > 65 °C pour une période, lors de laquelle le degré de dénaturation souhaité d'une valeur ≤ 25 % n'est pas dépassé ; et
- ajouter de l'eau afin d'obtenir un mélange aqueux avant ou après le chauffage, sachant que le mélange aqueux est ajusté, par l'ajout de l'eau, sur une teneur en substance sèche d'une valeur < 25 % en poids.

8. Procédé selon la revendication 7,
selon lequel l'extraction préalable est effectuée à une valeur de pH de l'eau d'une valeur < 5.

9. Procédé selon la revendication 7 ou 8,
selon lequel l'extraction est effectuée à une valeur de pH de l'eau < 7,0.

10. Procédé selon l'une quelconque des revendications 7 à 9,
selon lequel le mélange est transvasé dans un emballage, l'emballage est fermé de manière étanche aux liquides et le mélange situé dans l'emballage fermé est chauffé à une température d'une valeur > 110 °C.

11. Utilisation de la préparation à base de protéines selon l'une quelconque des revendications 1 à 5 en tant que substitut des protéines de lait, des protéines de l'oeuf ou en tant que protéine végétale sèche dans des produits alimentaires.
